# EUROPEAN PATENT APPLICATION

(11) **EP 2 436 413 A1**
(43) Date of publication of application: **04.04.2012**
(21) Application number: 11007214.7
(22) Date of filing: 06.09.2011
(51) Int. Cl.: A61M 5/50, A61M 5/315

(54) **Syringe assembly**

(30) Priority: 29.09.2010 US 893532
(71) Applicant: TYCO HEALTHCARE GROUP LP, Mansfield MA 02048 (US)
(72) Inventor: Preis, Colin, Hopkinton, MA 01748 (US)
(74) Representative: Gray, James

(57) **Abstract**

A syringe assembly includes a syringe body having an internal fluid reservoir and a fluid contained within the fluid reservoir and defining a fluid outlet adjacent the distal end thereof, a plunger at least partially disposed within the syringe body and having a plunger rod and a distal plunger head and being adapted for longitudinal movement from an initial retracted position to an actuated advanced position and a dispensing element disposed within the internal fluid reservoir of the syringe body and distal of the plunger head. The dispensing element is dimensioned and arranged to cooperate with the plunger head to permit engagement and advancing movement of the dispensing element during corresponding movement of the plunger head without effecting a coupling relationship with the plunger head whereby the plunger is movable toward the retracted position without causing corresponding movement of the dispensing element.

## Description

### BACKGROUND

### 1. Technical Field

The present disclosure generally relates to syringes and, in particular, relates to medical syringes such as disposable syringes and prefilled syringes, which may be discarded after a single administration.

### 2. Background

Single-use syringes are well known in the medical arts. A single-use syringe prevents reuse of the syringe to minimize exposure of patients to HIV, hepatitis and other blood-borne pathogens. A variety of different types of single-use syringes are known. These include syringes having frangible plunger disabling mechanisms and those having complex locking elements, e.g., precision metal stampings or the like. Those with complex locking mechanisms are expensive and require complicated manufacturing procedures. With regard to those syringes having disabling mechanisms, it has been difficult to engineer, design and manufacture such mechanisms to operate to eject fluid from a syringe while assuring disablement of the plunger during or after use.

Accordingly, a continuing need exists in the medical arts for a simple, reliable, robust single-use syringe.

### SUMMARY

In accordance with one embodiment of the present disclosure, a syringe assembly includes a syringe body having an internal fluid reservoir and a fluid contained within the fluid reservoir and defining a fluid outlet adjacent the distal end thereof, a plunger at least partially disposed within the syringe body and having a plunger rod and a distal plunger head and being adapted for longitudinal movement from an initial retracted position to an actuated advanced position, and a dispensing element disposed within the internal fluid reservoir of the syringe body and distal of the plunger head. The dispensing element is positioned to be engaged by the plunger head during movement of the plunger toward the advanced position thereof such that the dispensing element dispenses the fluid through the fluid outlet of the syringe body. The dispensing element is dimensioned and arranged to cooperate with the plunger head to permit engagement and advancing movement of the dispensing element during corresponding movement of the plunger head without effecting a coupling relationship with the plunger head whereby the plunger is movable toward the retracted position without causing corresponding movement of the dispensing element.

The dispensing element may include a plurality of longitudinally spaced external ribs whereby adjacent ribs define recesses therebetween. The ribs and recesses cooperate to reduce forces required to move the dispensing element within the syringe body. The dispensing element may define a substantially frusto-conical shaped distal face.

A conduit may be connected adjacent the distal end of the syringe body and may define a lumen in fluid communication with the internal fluid reservoir. The conduit may include a distal penetrating end.

In accordance with another embodiment, a syringe assembly includes a syringe body having an internal fluid reservoir and a fluid contained within the fluid reservoir and defining a fluid outlet adjacent the distal end thereof, a needled conduit connected adjacent the distal end of the syringe body and defining a lumen in fluid communication with the internal fluid reservoir, a plunger at least partially disposed within the syringe body and having a plunger rod and a distal plunger head and being adapted for longitudinal movement from an initial retracted position to an actuated advanced position, and a dispensing element disposed within the internal fluid reservoir of the syringe body distal of the plunger head. The dispensing element is positioned to be engaged by the plunger head during movement of the plunger toward the advanced position thereof such that the dispensing element dispenses the fluid through the fluid outlet of the syringe body and into the needled conduit. At least one of the dispensing element and the plunger head is devoid of irregularities to permit engagement and advancing movement of the dispensing element during corresponding movement of the plunger head without effecting a coupling relationship with the plunger head. In this manner, the plunger is movable toward the retracted position without causing corresponding movement of the dispensing element.

A surgical procedure is also provided. The surgical procedure includes the steps of:
providing a syringe assembly including a syringe body having an internal fluid reservoir, a plunger at least partially disposed within the syringe body and a dispensing element within the internal fluid reservoir distal of the plunger;
advancing the plunger relative to the syringe body from an initial position to an advanced position such that the plunger causes corresponding advancing movement of the dispensing element whereby the dispensing element expresses fluid from the internal fluid reservoir of the syringe body without; and
establishing a non coupling relationship between the plunger and the dispensing element such that subsequent to movement of the plunger to the advanced position, the plunger may be moved toward the initial position without causing corresponding withdrawal of the dispensing element thereby substantially preventing ingress of fluids within the internal fluid reservoir.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

**FIG. 1** is a side view of a syringe assembly illustrating the syringe body and the needle extending from the syringe body in accordance with the embodiments of the present disclosure;

**FIG. 2** is an exploded view of the syringe assembly of **FIG. 1****;**

**FIG. 3** is a perspective view of the syringe body of the syringe assembly of **FIG. 1****;**

**FIG. 4** is a perspective view of the syringe body of **FIG. 3** with a section of the syringe body removed, illustrating the dispensing element within the syringe body and the plunger;

**FIG. 5** is a side view of the dispensing element;

**FIGS. 6, 7,** and **8** are cross-sectional views illustrating movement of the plunger between a retracted position and an advanced position;

**FIG. 9** is an exploded view illustrating an alternate embodiment of the syringe assembly; and

**FIG. 10** is an exploded perspective view of another alternative embodiment of the syringe assembly.

### DETAILED DESCRIPTION OF THE PREFERRED EMBODIMENT(S)

Embodiments of the presently disclosed plunger and syringe assembly will now be described in detail with reference to the drawings wherein like reference numerals designate identical or corresponding elements in each of the several views. In this description, the term "proximal" or "trailing" is generally used to indicate the relative nearness of a referenced item to a clinician using the assembly and the term "distal" or "heading" is used to indicate the remoteness of a referenced item to a clinician using the device.

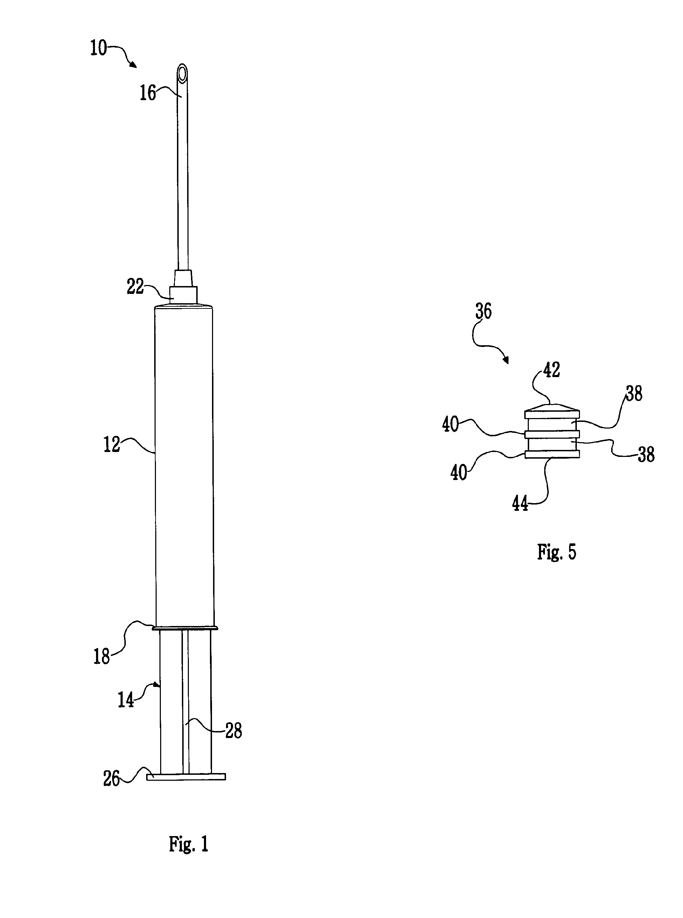

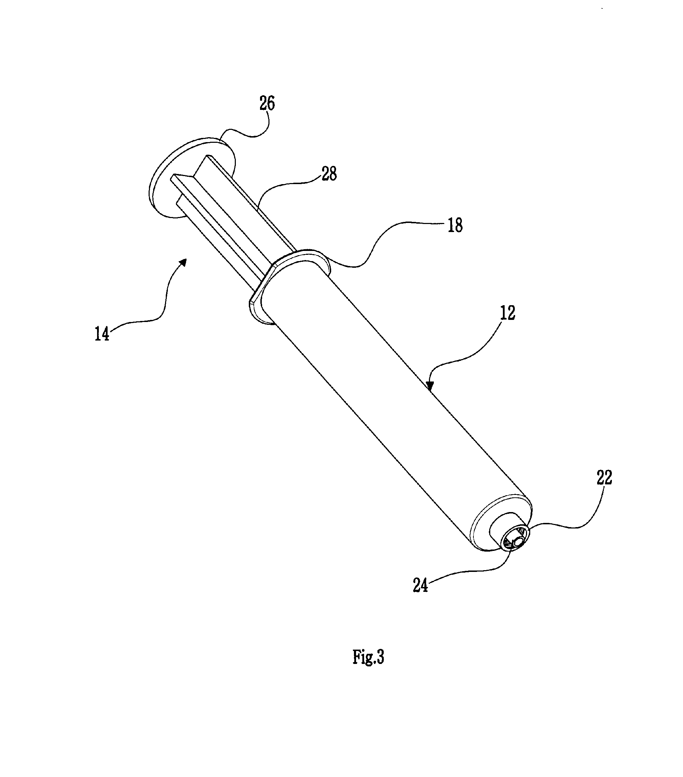

Referring now to the drawings wherein like components are designated by like reference numerals throughout the several views, **FIGS. 1-4** illustrate a syringe assembly **10** in accordance with the principles of the present disclosure. In one embodiment, syringe assembly **10** may be a single-use assembly, i.e., disposable after a single application.. In another embodiment, syringe assembly 10 may be a prefilled syringe assembly, in particular a prefilled sterilized syringe, for which the sterilized portion of the syringe assembly may not extend beyond a plunger tip into an unfilled portion of a syringe barrell. In certain applications, syringe assembly **10** may contain a flushing solution, e.g., saline or the like. Alternatively, syringe assembly **10** may contain medicinals, including antibiotics, pain medication, therapeutic drugs, heparin or the like.

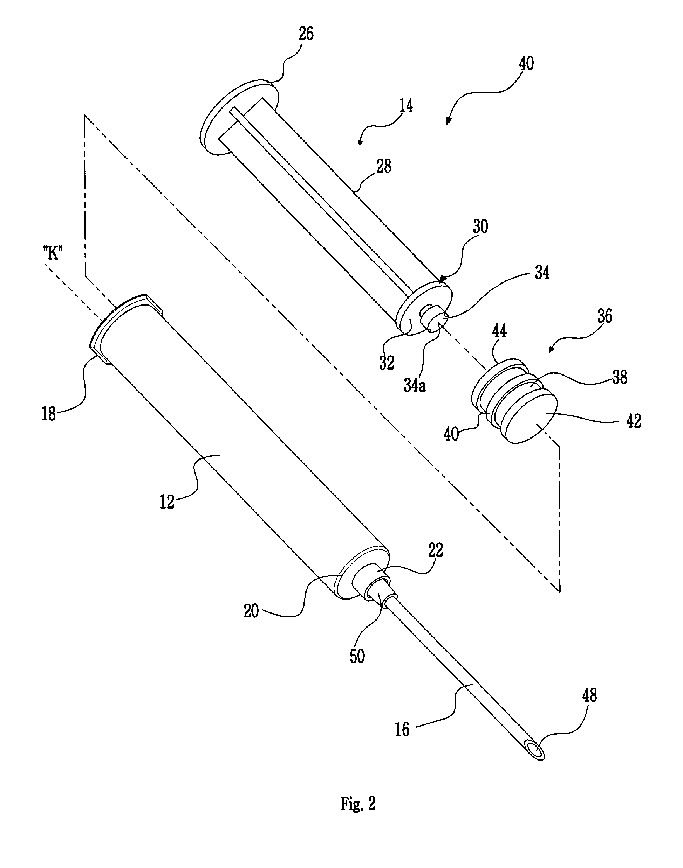

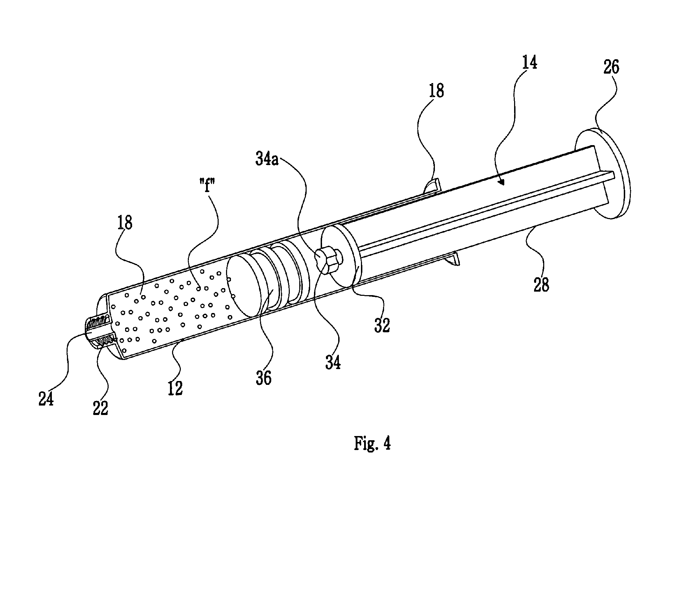

Syringe assembly **10** includes syringe body **12,** plunger **14** at least partially received within syringe body **12** and needle **16** which is mounted to syringe body **12.** As best depicted in **FIGS. 2-4****,** syringe body **12** defines longitudinal axis "K" extending a length of syringe body **12.** Syringe body **12** defines fluid chamber or internal reservoir **18** (see **FIG. 4****),** which contains pre-filled medicinals "f". Adjacent a proximal end of syringe body **12** is flange **18,** which may be integrally formed with syringe body **12.** Flange **18** may be relatively enlarged to enhance engagement by the clinician. Distal end **20** of syringe body **12** includes needle connector mechanism **22,** which may, in one embodiment, be a luer connector or luer lock. Distal end **20** further defines fluid outlet **24** which is in fluid communication with internal reservoir **18.**

Plunger **14** includes proximal flange **26** (see **FIGS. 2** and **4****),** elongated plunger rod **28** extending from the proximal flange **26** and plunger head **30** (see **FIGS.** 2 and **4****)** at a distal end of plunger rod **28.** Plunger flange **26** is enlarged to facilitate engagement by a clinician. Plunger rod **28** may have any cross-sectional dimension. In the embodiments depicted in **FIGS. 1-4****,** plunger rod **28** has a "+" shaped cross-section. Plunger head **30** includes enlarged disc **32** having a cross-section or diameter, which generally approximates an internal diameter defined by an internal wall of syringe body **12.** Plunger head **30** may further include protruding head segment **34** extending distally beyond enlarged disc **32.** Head segment **34** may define any configuration, but is dimensioned to provide an atraumatic distal or leading profile. In one embodiment, head segment **34** defines a substantially planar distal face **34**a. Head segment **34** may have other configurations including convex, concave or other shaped surface arrangements. It is further envisioned that plunger head **30** may be devoid of head segments **34,** and consist entirely of plunger disc **32.** Plunger head **30** may present a profile which is devoid of irregularities for reasons to be discussed hereinabove.

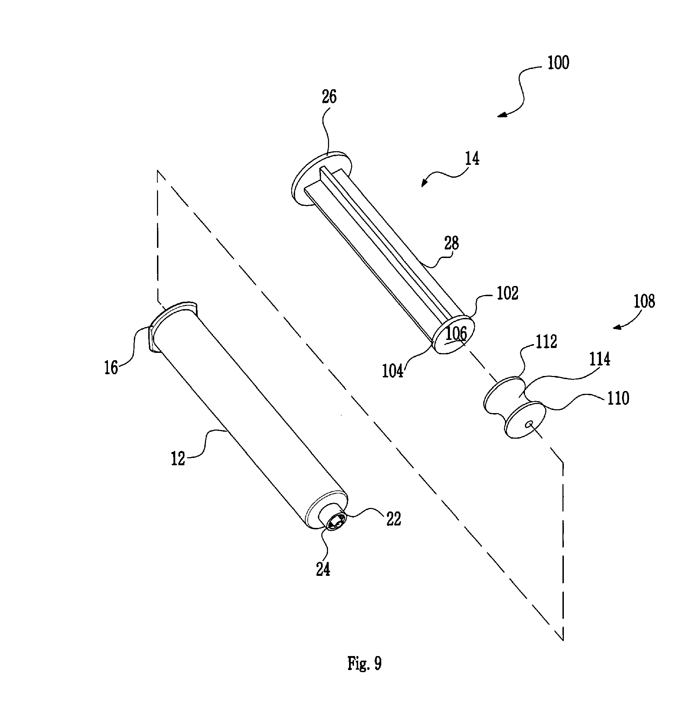

Referring to **FIGS. 4** and **5****,** syringe assembly **10** further includes expressing or dispensing element **36** disposed within the syringe body and abuting reservoir **18** of syringe body **12.** Dispensing element **36** may comprise an elastomeric material or the like. Dispensing element 36 defines, in one embodiment, a circular cross-sectional dimension, which generally approximates an inner dimension of syringe body **12.** In general, dispensing element **36** is adapted to traverse or longitudinally move within syringe reservoir **18** upon corresponding longitudinal movement of plunger **14** to dispense fluids through fluid outlet **24** of syringe body **12.** Dispensing element **36** may include annular recesses **38** (see **FIG. 9**) to reduce friction between dispensing element **36** and the internal wall of syringe body **12** to facilitate advancing movement. Annular recesses **38** are defined between adjacent ribs **40** of dispensing element **36.** Dispensing element **36** may include a leading or distal conical face **42,** which contacts the medicinal fluids within reservoir **18.**

With continued reference to **FIGS. 4** and **5****,** dispensing element **36** may include an atraumatic proximal or trailing face **44,** which is contacted by head segment **34** of plunger head **30** during advancement of plunger **14.** Trailing face **44** of dispensing element **36** may be planar or slightly arcuate, but is devoid of any recesses, depressions, undulations or other irregularities. In this manner, dispensing element **36** will not mechanically couple to, or with, plunger during any longitudinal movement of plunger **14.** In particular, during advancing or distal movement of plunger **14,** plunger head **30** including disc **32** and head segment **34,** may contact trailing face **44** of dispensing element **36** to cause corresponding advancing movement of dispensing element **36** and subsequent dispensing of pre-filled solution "f". However, trailing face **44** of dispensing element **36** will not couple with or become attached to plunger head **30.** Accordingly, plunger **14** may be retracted without causing any retracting movement of dispensing element **36,** i.e., dispensing element **36** will remain in an advanced position. More specifically, dispensing element **36** is dimensioned and arranged to cooperate with plunger head **30** to permit engagement and advancing movement of dispensing element **36** during corresponding movement of plunger head **30** without effecting a coupling relationship with plunger head **30,** whereby plunger **14** is movable toward the retracted position without causing corresponding movement of dispensing element **36.** This advantageously prevents use of syringe assembly **10** in any aspirating capacity, thereby preventing fluids to be drawn into syringe body **12.**

Referring again to **FIG. 1-2****,** needle or conduit **16** includes central lumen **46** which extends the length of the needle **16** and is in fluid communication with fluid outlet **24** of syringe body **12.** Needle **16** includes penetrating end **48** which is adapted to pierce tissue for entry within the vessel. Needle **16** further includes connector **50** adjacent its proximal end. Connector **50** couples with connector **22** of syringe body **12** to connect needle **16** to the syringe body **12.** Connector **50** may establish a releasable connection with connector **22** through a luer lock mechanism or the like, or may establish a more permanent connection with adhesives, cements, etc.

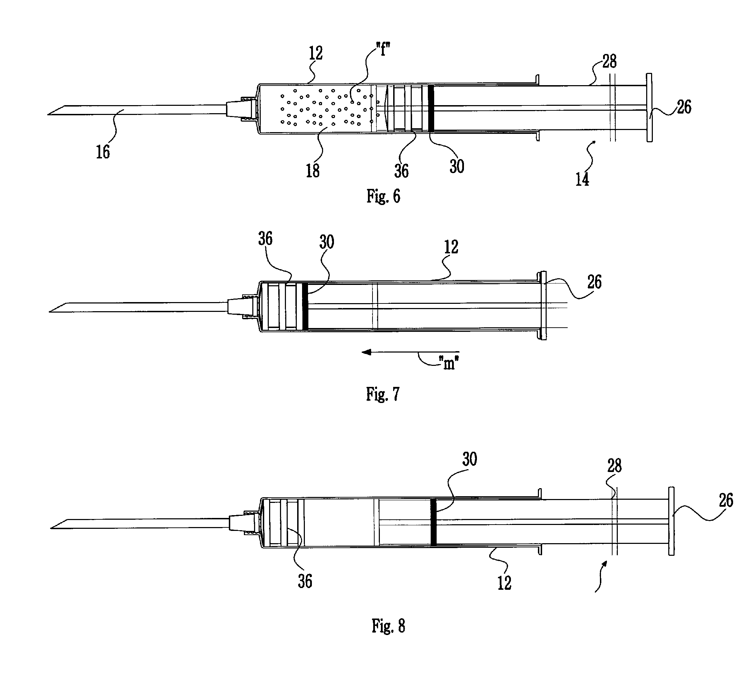

Referring to **FIGS. 6-8****,** cross-sectional views of syringe assembly **10** illustrating movement of plunger **14** within syringe body **12** are illustrated. As illustrated in **FIGS. 6-8****,** the embodiments of the present disclosure are constructed such that plunger **14** and dispensing element **36** cooperate to permit the dispensing element **36** to slidably move within reservoir **18** of syringe body **12.** In a first position, depicted in **FIG. 6****,** corresponding to a retracted position of plunger **14,** dispensing element **36** is positioned adjacent plunger head **30** of plunger **14,** i.e., the dispensing element **36** and plunger **14** are in a proximal most position with fluid "f" being within internal reservoir **18.** In **FIGS. 6-8****,** for illustrative purposes, plunger head **30** is shown without head segment **34.**

During advancement of plunger **14** within syringe body **12** in the direction of directional arrows "m" as depicted in **FIG. 7****,** plunger head **30** engages dispensing element **36** and causes corresponding advancement of the dispensing element **36.** Such movement of the dispensing element **36** causes the fluid "f' within internal reservoir **18** to be dispensed through fluid outlet **24** and into needle **16.** Plunger **14** is continually advanced to an actuated or fully advanced position, depicted in **FIG. 7****,** to correspondingly move dispensing element **36** to express the entire volume of fluids "f" from internal reservoir **18.**

As discussed hereinabove, during movement of the plunger **14,** plunger head **30** and dispensing element **36** do not cooperate to form an integrally functioning assembly, i.e., plunger **14** and dispensing element **36** are separate and distinct, non-couplable elements and are devoid of irregularities which would otherwise connect the components, so that retraction of plunger 14 prior or subsequent to dispensing all or a portion of the fluid from the internal reservoir does not also retract the dispensing element. In one embodiment, once dispensing element **36** is driven to the distal most location, plunger **14** may be retracted toward its initial position without causing corresponding movement of the dispensing element **36.** This substantially minimizes or removes the potential of syringe **10** to be used in an aspirating capacity, the benefits of which being hereinbelow discussed. Dispensing element **36** may be dimensioned to engage the internal wall of syringe body **12** in frictional relation therewith to be fixed in the distal position depicted in **FIG. 8****.** In one embodiment, dispensing element **36** occupies the entire cross-sectional volume of internal reservoir **18** such that any potential vacuum, suction or aspirating forces created during a retracting movement of plunger **14** (e.g., subsequent to expressing the fluids "f") in the opposite direction as also depicted in **FIG. 8** is not communicated beyond the dispensing element **36** to fluid outlet **34.**

Referring to **FIG. 9****,** an exploded perspective view of an alternate embodiment of a syringe assembly **100** is illustrated. In accordance with this embodiment, plunger head **102** includes a plunger disc **104.** Plunger disc **104** defines a leading or distal surface **106** which is devoid of irregularities. Dispensing element **108** defines leading (distal) and trailing (proximal) ends **110, 112** and central recessed segment **114.** Leading and trailing ends **110, 112** define a cross-sectional dimension approximating the internal dimension of syringe body **12.** Central recessed segment **114** reduces the level of force required to advance dispensing element **108** by minimizing the area of surface contact between the dispensing element **108** and the internal wall of syringe body **12.** Trailing end **112** of dispensing element **108** may also be devoid of irregularities to ensure that there is no coupling relation between the dispensing element **108** and plunger head **102.**

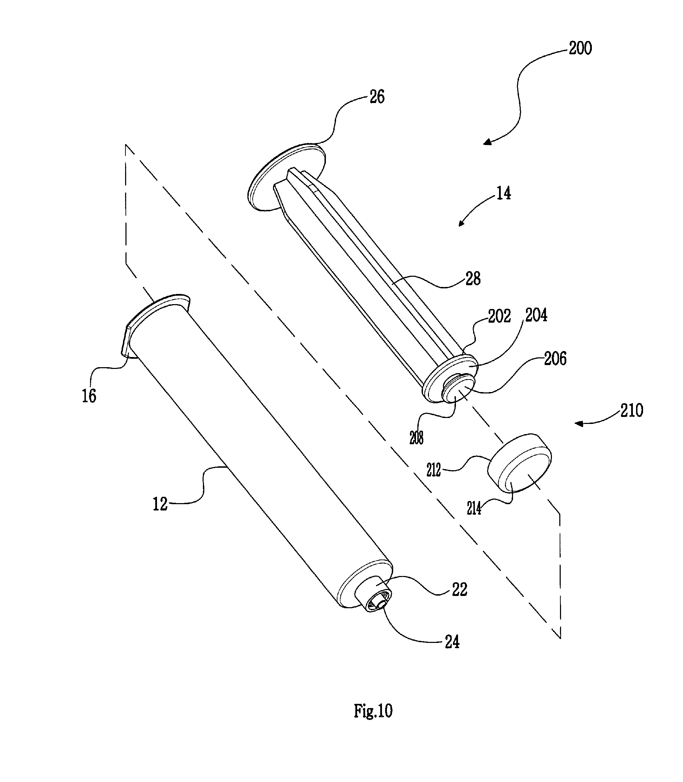

Referring to **FIG. 10****,** an exploded perspective view of another embodiment of the syringe assembly is depicted. Syringe assembly **200** includes plunger head **202** having first and second disc elements **204, 206.** Disc element **206** is the leading or distal disc and defines a smoothly planar end face **208.** Dispensing element **210** is substantially cylindrical and defines a corresponding proximal end face **212** which is devoid of irregularities and may have a similar profile to distal end face **214.** In this manner, dispensing element **210** will not couple with plunger head **202.**

The advantages of the example embodiments of the present disclosure may include at least providing assurance of clinical observance of indications for use for prefilled flush syringes. The indications for use for prefilled flush syringes are limited to the flushing of I.V. access devices for the purpose of maintaining the patency of those devices. Clinicians sometimes use saline flush syringes to reconstitute lyophilized drugs. Clinicians perform such tasks by expressing the contents of the prefilled syringe into the vial containing the lyophilized drug. The clinicians aspirate the reconstituted drug back into the syringe for subsequent delivery to the patient. This is an off-label use and is not recommended.

The example embodiments of the present disclosure prevent the clinicians from using the same syringe for dispensing the solution into the drug vial and aspirating the reconstituted drug for delivery. By eliminating the apparent convenience of being able to use the syringe for all operations related to reconstituting and delivering a lyophilized drug, a clinician may be significantly discouraged from using the flush solution syringe for such application. The example embodiments of the present disclosure thus effectively prevent clinicians from reusing syringes for any purpose once the initial prefilled solution is expressed. In essence, the syringe has an auto-disable feature once the original solution has been expressed. Another advantage of some embodiments of the present invention is that the clinician is prevented from pulling back the plunger rod of a sterilized prefilled syringe into a non-sterile portion of the syringe barrel, thereby reducing or eliminating contamination during use and administration of the sterilized contents of the prefilled syringe.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure.

## Claims

1. A syringe assembly, which comprises:
a syringe body having an internal fluid reservoir and a fluid contained within the fluid reservoir, the syringe body defining a longitudinal axis, and proximal and distal ends, the syringe body defining a fluid outlet adjacent the distal end thereof;
a plunger at least partially disposed within the syringe body, the plunger including a plunger rod and a distal plunger head, the plunger adapted for longitudinal movement from an initial retracted position to an actuated advanced position; and
a dispensing element disposed within the syringe body and abutting the internal fluid reservoir of the syringe body and distal of the plunger head, the dispensing element positioned to be engaged by the plunger head during movement of the plunger toward the advanced position thereof such that the dispensing element dispenses the fluid through the fluid outlet of the syringe body, the dispensing element being dimensioned and arranged to cooperate with the plunger head to permit engagement and advancing movement of the dispensing element during corresponding movement of the plunger head without effecting a coupling relationship with the plunger head whereby the plunger is movable toward the retracted position without causing corresponding movement of the dispensing element.

2. The syringe assembly according to claim 1, wherein the dispensing element includes a plurality of longitudinally spaced external ribs, whereby adjacent ribs define recesses therebetween, the ribs and recesses cooperating to reduce forces required to move the dispensing element within the syringe body.

3. The syringe assembly according to claim **1,** wherein the dispensing element defines a substantially frusto-conical shaped distal face.

4. The syringe assembly according to claim **1,** including a conduit connected adjacent the distal end of the syringe body and defining a lumen in fluid communication with the internal fluid reservoir.

5. The syringe assembly according to claim **4,** wherein the conduit includes a distal penetrating end.

6. The syringe assembly according to claim **1,** wherein the plunger head defines a substantially planar distal surface portion and the dispensing element defines a substantially planar proximal surface portion, the substantially planar surface portions cooperating without effecting the coupling relationship.

7. The syringe assembly according to claim **1,** wherein the plunger head is a disc member, the disc member having a protrusion depending therefrom, the protrusion cooperating with the dispensing element without effecting a coupling relationship.

8. A syringe assembly, which comprises:
a syringe body having an internal fluid reservoir and a fluid contained within the fluid reservoir, the syringe body defining a longitudinal axis, and proximal and distal ends, the syringe body defining a fluid outlet adjacent the distal end thereof;
a needled conduit connected adjacent the distal end of the syringe body and defining a lumen in fluid communication with the internal fluid reservoir.
a plunger at least partially disposed within the syringe body, the plunger including a plunger rod and a distal plunger head, the plunger adapted for longitudinal movement from an initial retracted position to an actuated advanced position; and
a dispensing element disposed within the syringe body and abutting the internal fluid reservoir of the syringe body and distal of the plunger head, the dispensing element positioned to be engaged by the plunger head during movement of the plunger toward the advanced position thereof such that the dispensing element dispenses the fluid through the fluid outlet of the syringe body and into the needled conduit, at least one of the dispensing element and the plunger head being devoid of irregularities to permit engagement and advancing movement of the dispensing element during corresponding movement of the plunger head without effecting a coupling relationship with the plunger head whereby the plunger is movable toward the retracted position without causing corresponding movement of the dispensing element.

9. A surgical procedure, comprising the steps of:
providing a syringe assembly including a syringe body having an internal fluid reservoir, a plunger at least partially disposed within the syringe body and a dispensing element within the internal fluid reservoir distal of the plunger;
advancing the plunger relative to the syringe body from an initial position to an advanced position such that the plunger causes corresponding advancing movement of the dispensing element whereby the dispensing element expresses fluid from the internal fluid reservoir of the syringe body without; and
establishing a non coupling relationship between the plunger and the dispensing element such that subsequent to movement of the plunger to the advanced position, the plunger may be moved toward the initial position without causing corresponding withdrawal of the dispensing element thereby substantially preventing ingress of fluids within the internal fluid reservoir.
